# EUROPEAN PATENT APPLICATION

(11) **EP 4 393 499 A1**
(43) Date of publication of application: **03.07.2024**
(21) Application number: 22383291.6
(22) Date of filing: 27.12.2022
(51) Int. Cl.: A61K 35/28, C07K 14/54, C07K 14/705

(54) **SMALL EXTRACELLULAR VESICLES WITH ANTIFIBROTIC PROPERTIES**

(71) Applicant: Fundación Para la Investigación del Hospital Universitario y Politécnico La Fe de la Comunidad Valenciana, 46026 Valencia (ES)
(72) Inventor: Tejedor Gascón, Sandra, VALENCIA (ES); González-King Garibotti, Hernán, VALENCIA (ES); Buigues Caravaca, Marc, VALENCIA (ES); García, Nahuel Aquiles, Valencia (ES); Sepúlveda Sanchis, Pilar, Valencia (ES)
(74) Representative: Herrero & Asociados, S.L.

(57) **Abstract**

The present invention refers to an isolated Small Extracellular Vesicle (SEV) with a mutated form of functional Oncostatin M expressed on its surface. Further, pharmaceutical compositions comprising one or more SEVs, as active ingredient, are also contemplated. The invention also refers to an in vitro method for producing the SEVs of the invention, comprising the steps of a) immortalizing a human MSC line by telomerase overexpression, b) introducing in the immortalized MSCs a vector expression constituted by a genetic construct, comprising the modified OSM sequence, selected from SEQ ID NO 1 or SEQ ID NO 2, fused to CD81 sequence, and an inducible promoter, which responds to doxycycline, c) culturing the genetically modified MSCs obtained in b) in a medium culture under conditions permitting their expansion, d) adding doxocycline to the culture medium, and e) Collecting Small Extracellular vesicles (SEVs) from the culture supernatant.

## Description

### Field of the invention

The present invention relates generally to the Health Sector, and more particular to the prevention and treatment of Fibrosis. Specifically, the invention relates to nanovesicles, particularly a subpopulation called Small Extracellular Vesicles (SEVs), derived from mesenchymal stromal cells genetically modified, with enhanced therapeutic potential by the overexpression of OSM on their surface. The invention also contemplates the method for obtaining said SEVs, pharmaceuticals compositions comprising said SEVs, and clinical applications thereof in the treatment of profibrotic diseases, in particular those related to cardiac fibrosis.

### Background of the invention

There are currently a number of pathologies related to the unwanted or uncontrolled proliferation of fibroblasts for which there is no effective solution, such as acute myocardial infarction or pulmonary fibrosis.

In particular, myocardial interstitial fibrosis is a pathological event associated to an excessive extracellular matrix (ECM) deposition that induces morphological and functional alterations in myocardial tissue architecture. Activated fibroblasts lead to differentiation into myofibroblasts and promote fibrosis while tissue-resident macrophages have been shown to prevent it (Chakarov, S. et al. Two distinct interstitial macrophage populations coexist across tissues in specific subtissular niches. Science (1979) 363, (2019*).* The contribution of these populations to cardiac healing is dynamic during the progression of disease. Initial ECM deposition by activated fibroblasts is a protective mechanism and can be beneficial for wound healing and tissue regeneration. However, when this spatiotemporal event is not resolved, there is a progression to tissue remodelling and heart failure (HF) (Jellis, C., Martin, J., Narula, J. & Marwick, T. H. Assessment of nonischemic myocardial fibrosis. J Am Coll Cardiol 56, 89-97 (2010*).* Hence, new therapeutic strategies to modulate the accumulation of interstitial fibrosis are still needed.

In this context, nanovesicles and, more particularly, small extracellular vesicles (SEVs), secreted by mesenchymal stromal cells (MSCs), have emerged as a promising tool to improve cardiac function by modulating apoptosis, ROS generation, angiogenesis, fibrosis, and inflammation (Arslan, F. et al. Mesenchymal stem cell-derived exosomes increase ATP levels, decrease oxidative stress and activate PI3K/Akt pathway to enhance myocardial viability and prevent adverse remodeling after myocardial ischemia/reperfusion injury. Stem Cell Res 10, 301-312 (2013*);* Maacha, S. et al. Paracrine Mechanisms of Mesenchymal Stromal Cells in Angiogenesis. Stem Cells Int 2020, (2020*);* Kordelas, L. et al. MSC-derived exosomes: a novel tool to treat therapy-refractory graft-versus-host disease. Leukemia 28, 970-973 (2014*);* Amable, P. R., Teixeira, M. V. T., Carias, R. B. V., Granjeiro, J. M. & Borojevic, R. Protein synthesis and secretion in human mesenchymal cells derived from bone marrow, adipose tissue and Wharton's jelly. Stem Cell Res Ther 5, (2014*);* Eirin, A. et al. MicroRNA and mRNA cargo of extracellular vesicles from porcine adipose tissue-derived mesenchymal stem cells. Gene 551, 55-64 (2014*);* Mirotsou, M., Jayawardena, T. M., Schmeckpeper, J., Gnecchi, M. & Dzau, V. J. Paracrine mechanisms of stem cell reparative and regenerative actions in the heart. J Mol Cell Cardiol 50, 280-289 (2011*);* Sánchez-Sánchez, R. et al. miR-4732-3p in Extracellular Vesicles From Mesenchymal Stromal Cells Is Cardioprotective During Myocardial Ischemia. Front Cell Dev Biol 9, (2021*).* Also SEVs included in the secretome of human cardiac stem cells (CSC) were able to reduce fibrotic scar tissue, interstitial fibrosis, cardiomyocyte hypertrophy after subcutaneous implantation of a device encapsulating CSC in infarcted rats *(*Kompa, A. R. et al. Sustained subcutaneous delivery of secretome of human cardiac stem cells promotes cardiac repair following myocardial infarction. Cardiovasc Res 117, 918-929 (2021*).* SEVs have an intrinsic similarity to cell membranes, a unique biomolecules profile, deformable cytoskeleton (Hood, J. L. & Wickline, S. A. A systematic approach to exosome-based translational nanomedicine. Wiley Interdiscip Rev Nanomed Nanobiotechnol 4, 458-467 (2012*), slightly negative zeta potential for long circulation;* Malhotra, H. et al. Exosomes: Tunable Nano Vehicles for Macromolecular Delivery of Transferrin and Lactoferrin to Specific Intracellular Compartment. J Biomed Nanotechnol 12, 1101-1114 (2016*);* Rupert, D. L. M., Claudio, V., Lässer, C. & Bally, M. Methods for the physical characterization and quantification of extracellular vesicles in biological samples. Biochim Biophys Acta Gen Subj 1861, 3164-3179 (2017*),* biocompatibility (Yang, Y., Hong, Y., Cho, E., Kim, G. B. & Kim, I. S. Extracellular vesicles as a platform for membrane-associated therapeutic protein delivery. J Extracell Vesicles 7, (2018*)),* small size for penetration into deep tissues (Vader, P., Mol, E. A., Pasterkamp, G. & Schiffelers, R. M. Extracellular vesicles for drug delivery. Adv Drug Deliv Rev 106, 148-156 (2016*)* and possibility to be modified to augment their therapeutic activity. These interesting properties have increased the scientific community interest for their use in cell-free based therapies for several clinical applications, such as cardiac repair. However, to date only one clinical trial using SEVs is recruiting patients with acute myocardial infarction (AMI)(*StudyRecord*|*BetaClinicalTrials.gov.https:*//*beta.clinicaltrials.gov*/*study*/*NCT04327635).*

Oncostatin M (OSM), a member of the interleukin 6 cytokine family, is a pleiotropic cytokine secreted by T cells, monocytes, macrophages, dendritic cells, and neutrophils in response to hypoxia, as it is regulated by hypoxia inducible factor (HIF-1 *α*) (Abe, H. et al. Macrophage hypoxia signaling regulates cardiac fibrosis via Oncostatin M. Nature Communications 2019 10:1 10, 1-10 (2019*);* Nair, B. C. etal. Identification of a major growth factor for AIDS-Kaposi's sarcoma cells as oncostatin M. Science 255, 1430-1432 (1992*);* Zarling, J. M. et al. Oncostatin M: a growth regulator produced by differentiated histiocytic lymphoma cells. Proc Natl Acad Sci U S A 83, 9739-9743 (1986*)*.

At a structural level, human OSM is initially formed by 252 amino acids and organized in three sections: signal peptide (SP), main chain and propeptide (PP). After translation, SP and PP are excised by proteolytic cleavage inside the cells producing mature OSM that is secreted into the extracellular space. Once in the extracellular space, this cytokine can interact with two heterodimeric receptor complexes: Type I and Type II. The Type I receptor complex is formed by leukemia inhibitory factor receptor (LIF-R) and glycoprotein 130 (gp130 or IL-31RA); while Type II receptor complex consists of the OSM receptor (IL-31RB) and IL-31RA (Adrian-Segarra, J. M. et al. The AB loop and D-helix in binding site III of human Oncostatin M (OSM) are required for OSM receptor activation. J Biol Chem 293, 7017-7029 (2018*);* Heinrich, P. C., Behrmann, I., Müller-Newen, G., Schaper, F. & Graeve, L. Interleukin-6-type cytokine signalling through the gp130/Jak/STAT pathway. Biochem J 334 ( Pt 2), 297-314 (1998*)*; Mosley, B. et al. Dual oncostatin M (OSM) receptors. Cloning and characterization of an alternative signaling subunit conferring OSM-specific receptor activation. J Biol Chem 271, 32635-32643 (1996*)*. This interaction induces the activation of Janus Kinase (JAK) family members (JAK1-2 and TYK2) through tyrosine phosphorylation, which induce the activation and nuclear translocation of STAT proteins (STAT1, 3, 5A and B) in receptor cells. Attending to the cardiovascular system, OSM has been related to proliferation, chemotaxis and tube formation in human microvascular endothelial cells *(*Vasse, M. et al. Oncostatin M induces angiogenesis in vitro and in vivo. Arterioscler Thromb Vasc Biol 19, 1835-1842 (1999*)*; Wijelath, E. S. et al. Oncostatin M induces basic fibroblast growth factor expression in endothelial cells and promotes endothelial cell proliferation, migration and spindle morphology. J Cell Sci 110 (Pt 7), 871-879 (1997); Pourtau, J. et al. Cyclooxygenase-2 activity is necessary for the angiogenic properties of oncostatin M. FEBS Lett 459, 453-457 (1999*)* and its delivery enhanced revascularization and tissue regeneration in a mouse model of acute muscle injury *(*Latroche, C. et al. Coupling between Myogenesis and Angiogenesis during Skeletal Muscle Regeneration Is Stimulated by Restorative Macrophages. Stem Cell Reports 9, 2018-2033 (2017*)).*

In addition to this, it has been described a role of OSM promoting CM dedifferentiation and cardiac tissue recovery after cardiac injury (Kubin, T. et al. Oncostatin M is a major mediator of cardiomyocyte dedifferentiation and remodeling. Cell Stem Cell 9, 420-432 (2011*);* Pöling, J. et al. The Janus face of OSM-mediated cardiomyocyte dedifferentiation during cardiac repair and disease. Cell Cycle 11, 439-445 (2012*).*

More importantly, although OSM is a proinflammatory cytokine that promotes fibrosis in different tissues, including liver and lung (Matsuda, M. et al. Oncostatin M causes liver fibrosis by regulating cooperation between hepatic stellate cells and macrophages in mice. Hepatology 67, 296-312 (2018*);* Mozaffarian, A. et al. Mechanisms of oncostatin M-induced pulmonary inflammation and fibrosis. J Immunol 181, 7243-7253 (2008*)),* during cardiac remodelling after a ischemic insult, the OSM secreted by Ly6Chi monocytes/macrophages suppresses cardiac fibroblast activation by means of inhibiting transforming growth factor beta 1 (TGF- *β* 1) *(*Abe, H. et al. Macrophage hypoxia signaling regulates cardiac fibrosis via Oncostatin M. Nature Communications 2019 10:1 10, 1-10 (2019*)* and the modulation of extracellular matrix deposition *(*Weiss, T. W. et al. The gp130 ligand oncostatin M regulates tissue inhibitor of metalloproteinases-1 through ERK1/2 and p38 in human adult cardiac myocytes and in human adult cardiac fibroblasts: a possible role for the gp 130/gp 130 ligand system in the modulation of extracellular matrix degradation in the human heart. J Mol Cell Cardiol 39, 545-551 (2005*).* Indeed, the role of OSM and its receptor complexes in the context of cardio protection, regeneration and failure has been recently reviewed *(*Kubin, T. et al. The Role of Oncostatin M and Its Receptor Complexes in Cardiomyocyte Protection, Regeneration, and Failure. International Journal of Molecular Sciences 2022, Vol. 23, Page 1811 23, 1811 (2022*).*

Based on the above, after an important research work, the authors of the present invention have obtained nanovesicles, in particular, small extracellular vesicles, that express functional OSM on their surface. They have demonstrated that the enrichment in functional OSM on SEVs surface increases their therapeutic potency in terms of anti-fibrotic properties both *in vivo* and *in vitro.*

### Brief description of the drawings

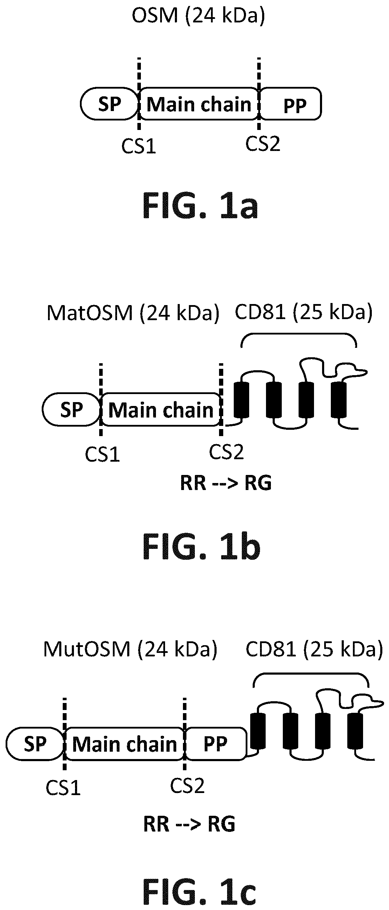

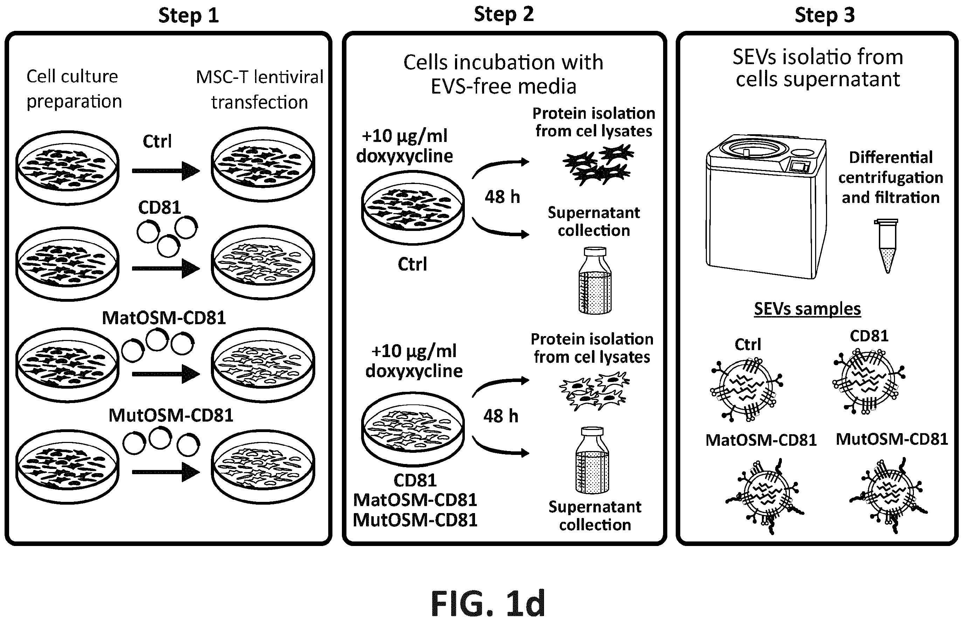

**Figure 1****. Generation of OSM-loaded MSC-T-SEVs by OSM specific sequence modification and fusion to CD81. (a-c)** Schematic representation of native OSM structure and recombinant proteins' structure with OSM introduced variants. **(a)** OSM protein native structure , **(b)** matureOSM (matOSM):The propeptide domain sequence has been removed and a nucleotide mutation to change an R for a G in CS2 has been introduced to avoid excision of matOSM from SEVs membrane, **(c)** mutantOSM (mutOSM) The whole OSM sequence has been maintained, but the same mutation to change R for a G in CS2 has been introduced. Both matOSM and mutOSM sequences have been anchored to CD81 tetraspanin to favour their presence on SEVs membrane. **(d)** Shows general experimental procedure to obtain engineered MSC-T and SEVs samples. **Step 1:** Cell culture preparation for lentiviral transfection. Lentivirus containing CD81, matOSM-CD81 or mutOSM-CD81 recombinant proteins were used to generate engineered MSC-T. **Step 2:** MSC-T expansion and incubation with EVs-free media containing doxycycline for 48h for supernatant collection and protein isolation form cell lysates. **Step 3:** SEVs isolation from cell supernatant by ultracentrifuge and filtration. Fusion of one protein with another is represented with a dash (-). OSM: Oncostatin M; SP: Signal peptide; CS: Cleavage site; PP: propeptide; R: Arginine; G: Glycine: MSC-T: immortalised mesenchymal stromal cells; EVs: extracellular vesicles; SEVs: small extracellular vesicles. Image has been created using BioRender.

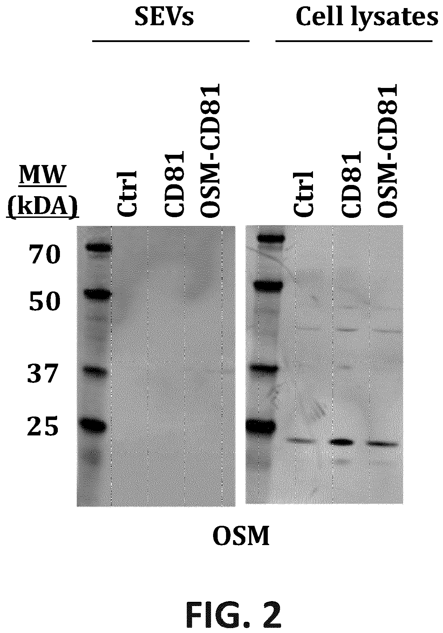

**Figure 2****. OSM protein detection on SEVs and cell lysates samples from Expi293F.** Expi293F were transiently transfected with lentiviral plasmids for CD81 and OSM-CD81 overexpression, and OSM protein was evaluated by Western Blot. SEVs and protein samples from non-transfected Expi293F cells were used as control (ctrl). Fusion of one protein with another is represented with a dash (-). Representative Western Blot membranes for each experimental condition is shown.

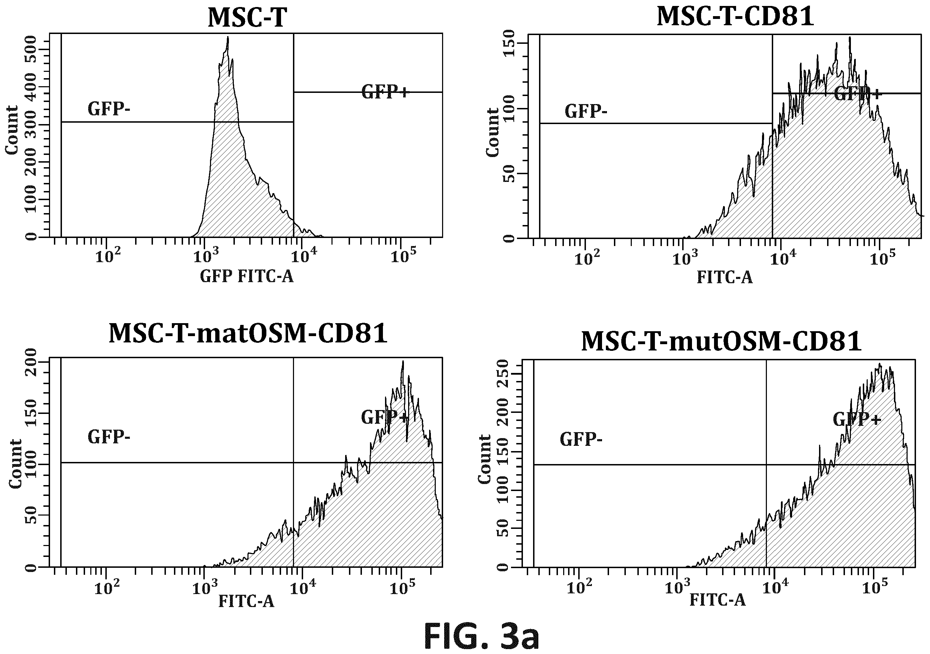

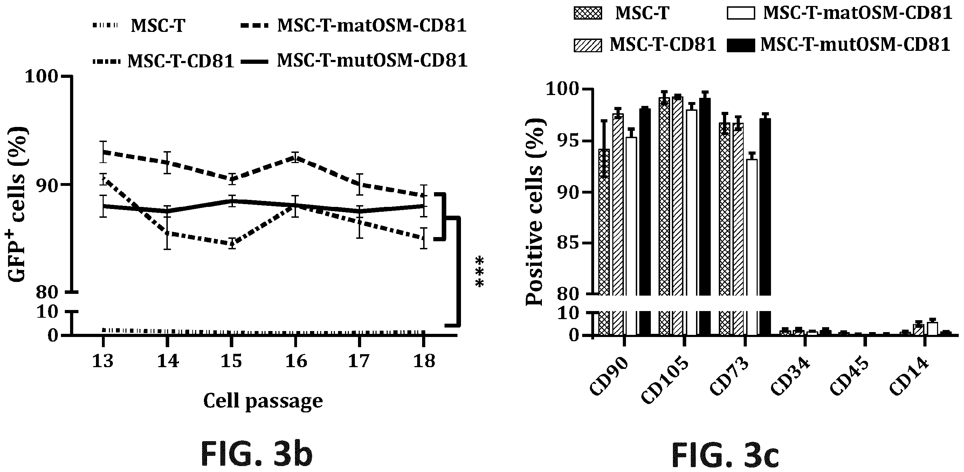

**Figure 3****. Genetically modified MSC-T generation and characterization. (a)** Representative histograms showing MSC-T GFP+ positive cells after CD81, matOSM-CD81 or mutOSM-CD81 plasmids introduction compared to control MSC-T. **(b)** Percentage of GFP+ cells over cell passages for MSC-T and genetically modified MSC-T measured by flow cytometry. **(c)** MSC-T and genetically modified MSC-T markers profile measured by flow cytometry. Percentage of positive cells for CD90, CD105, CD73, CD34, CD45 and CD14 on each cell type is shown. Fusion of OSM with CD81 is represented with a dash (-). Data corresponds to three independent experiments. Graphs show mean±SEM. Two-way ANOVA statistical test was used to compare data obtained from MSC-T with data obtained from MSC-T-CD81, MSC-T-matOSM-CD81 and MSC-T-mutOSM-CD81 (***p<0.001).

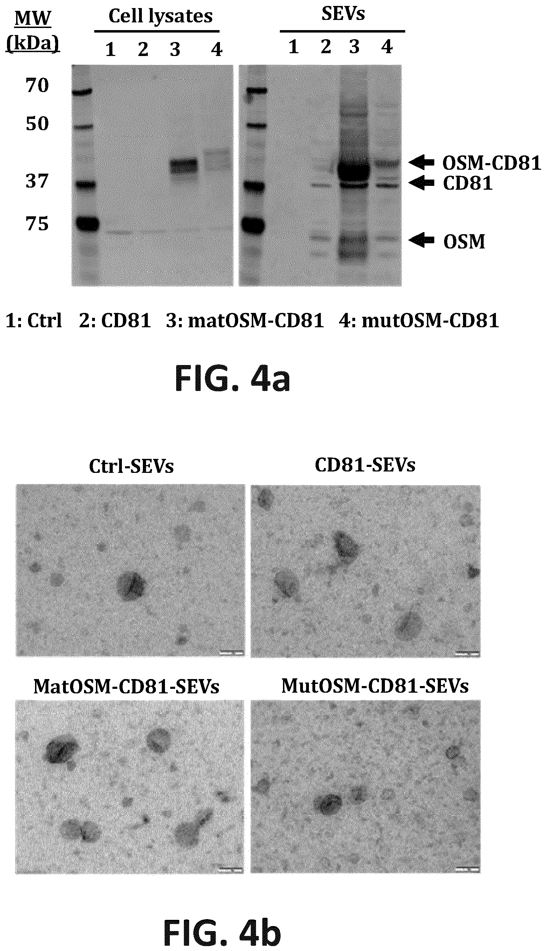

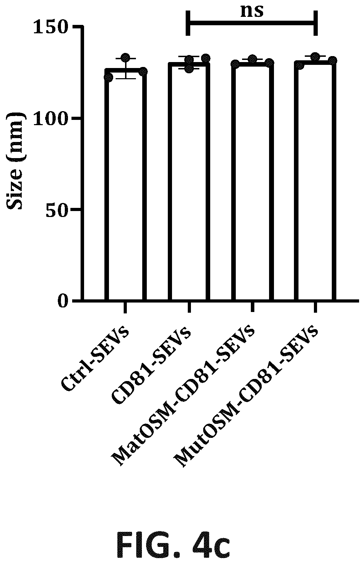

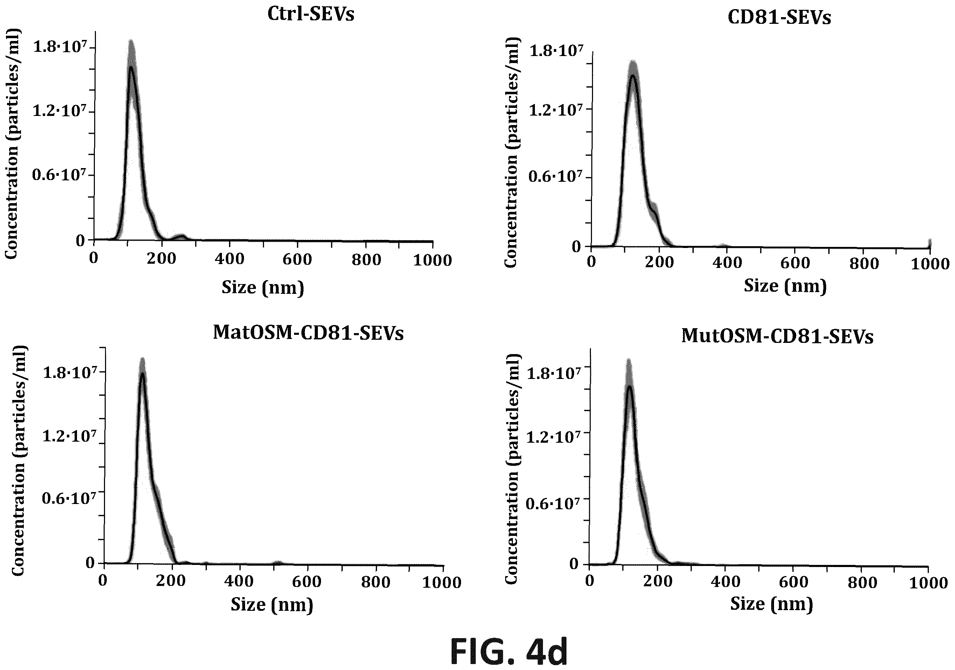

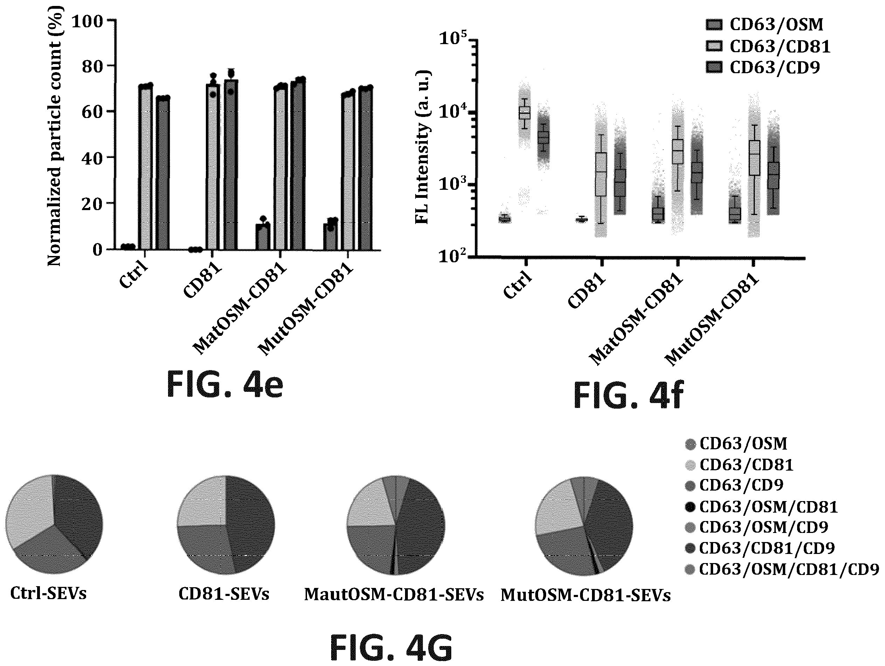

**Figure 4****. SEVs derived from genetically modified MSC-T express the recombinant proteins mat/mutOSM-CD81, maintaining the same physical characteristics in terms of morphology and size distribution. (a)** OSM detection in MSC-T (Ctrl) (lane 1), MSC-T-CD81 (CD81) (lane 2), MSC-T-matureOSM-CD81 (matOSM-CD81) (lane 3) and MSC-T-mutantOSM-CD81 (mutOSM-CD81) (lane 4) in cell lysates or SEVs protein samples. OSM presence was identified at both native molecular weight (22 to 24 kDa) and its expected molecular weight after fusion to CD81 (45 to 47 kDa). SEVs protein extracts: 10 µg/lane; Cell lysates protein extracts: 30 µg/lane. **(b)** Representative pictures of SEVs obtained by transmission electron microscopy analysis (scale bar = 100 nm). **(c-d)** Nanoparticle tracking analysis (NTA) results, showing particles size (nm) as a mean or in histogram plots. **(e)** Exoview analysis showing percentage of OSM, CD81 or CD9 positive SEVs in different samples after capture with CD63 antibody. **(f)** Fluorescence intensity in arbitrary units of SEVs double positive for CD63 and OSM, CD81 and CD9 in the different experimental groups. **(g)** The proportion of SEV positive for CD63 and some of the other markers (OSM, CD81, CD9) and also double or triple positive for these markers was analyzed in the 4 SEV experimental groups and ploted as pie charts. Each experiment was performed three times, and data were analyzed with ANOVA and Tukey's post-hoc test using values from ctrl cells or SEVs as reference condition and represented as mean ± SEM (ns: non-significant statistic differences).

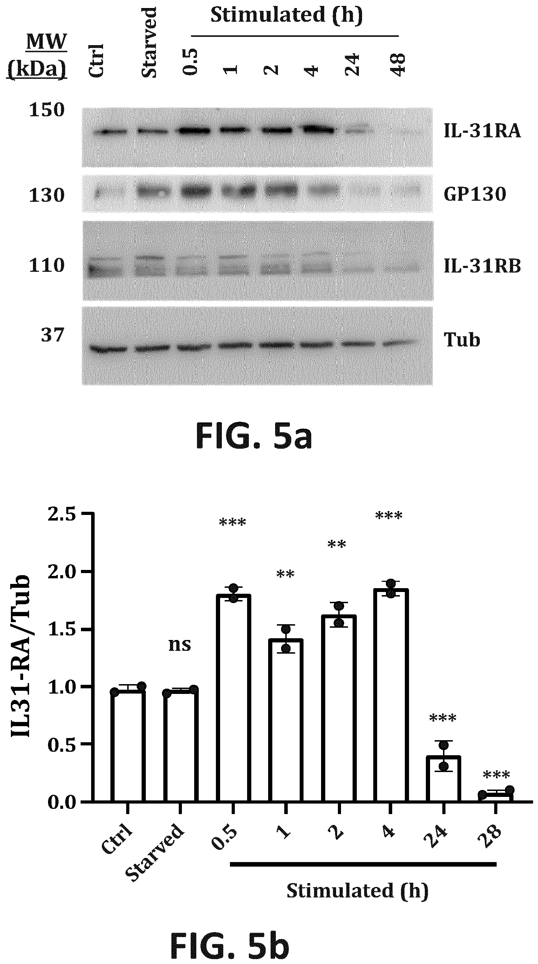

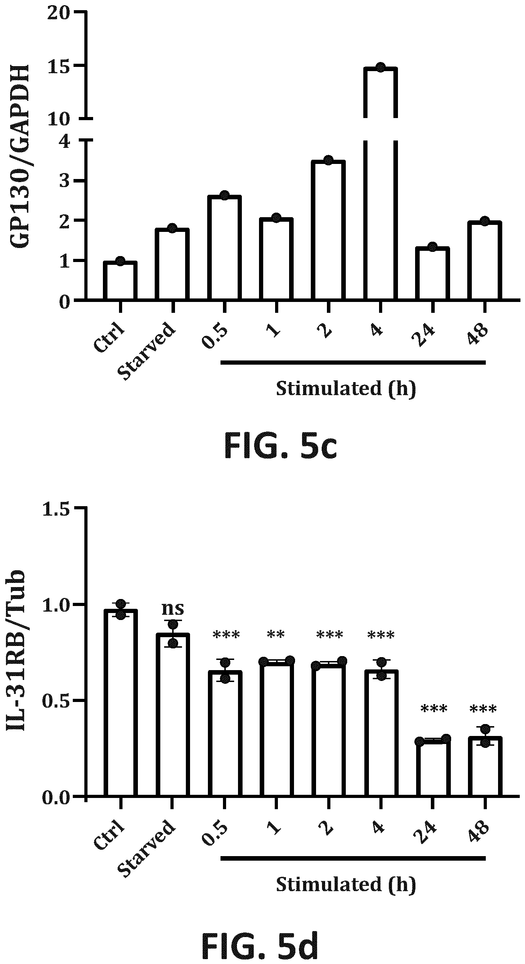

**Figure 5****. OSM receptors and IL-31 RA and GP130 are increased after HCF-V stimulation** in vitro. (a) Representative Western Blot for IL-31RA , GP130, IL-31RB and *β* -Tubulin (Tub) protein expression measured in basal conditions (ctrl), after starvation (starved) and after starvation and HCF-V stimulation using a pro-fibrotic cocktail containing L-ascorbic acid 2-phosphate, dextran sulphate and recombinant TGF *β*-1. Samples were collected both after starvation and after 0.5, 1, 2, 4, 24 and 48 h of starvation and stimulation. Untreated cells in basal conditions were used as control. **(b)** IL-31RA protein expression quantification. **(c)** GP130 protein expression quantification **(d)** IL-31RB protein expression quantification. Protein content was measured by densitometry in imaged software using Tubulin as loading control for each condition. Results were obtained from two independent experiments and were analyzed with ANOVA and Tukey's post-hoc test using values from ctrl cells as reference condition and represented as mean ± SEM (ns: non-significant statistic differences).

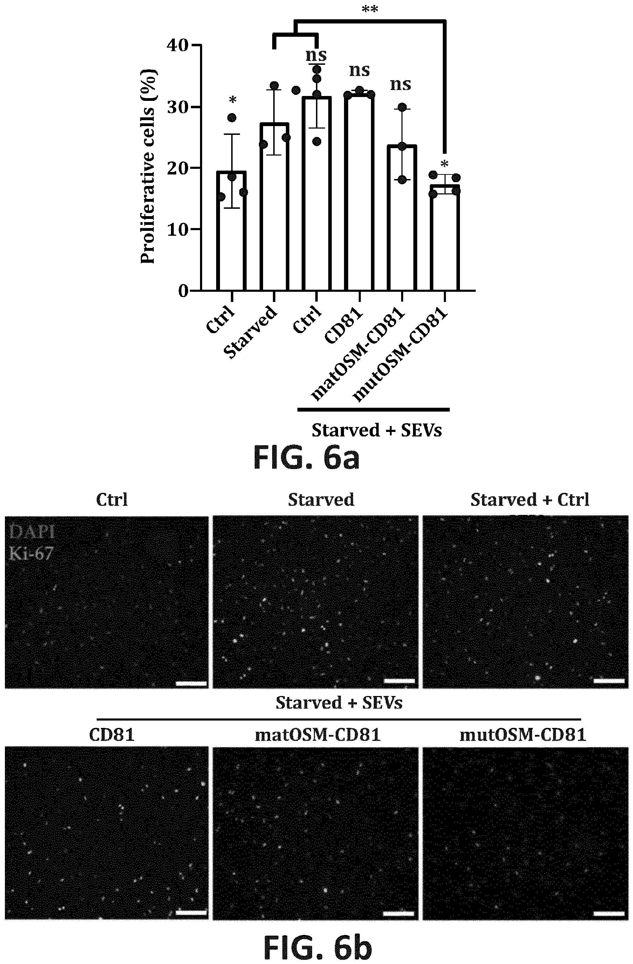

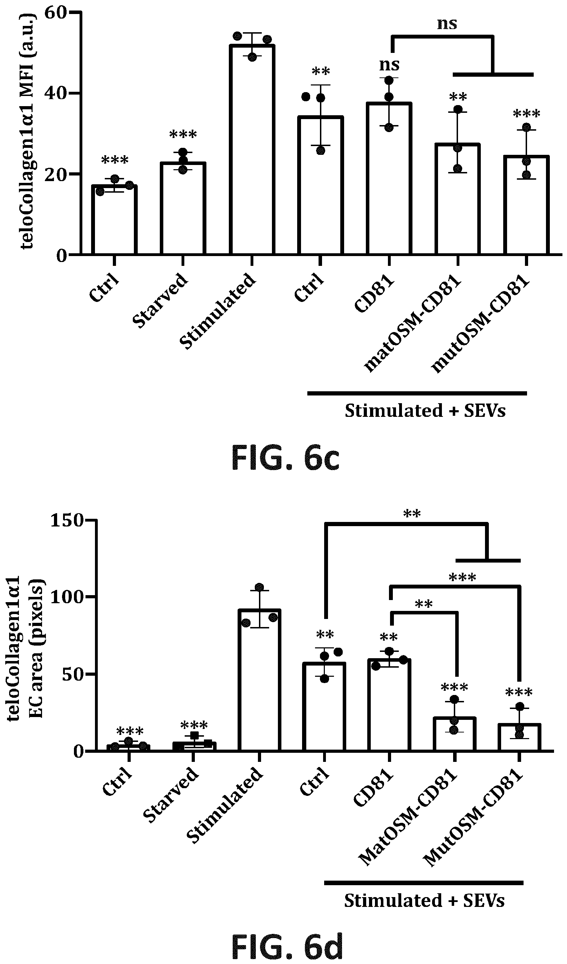

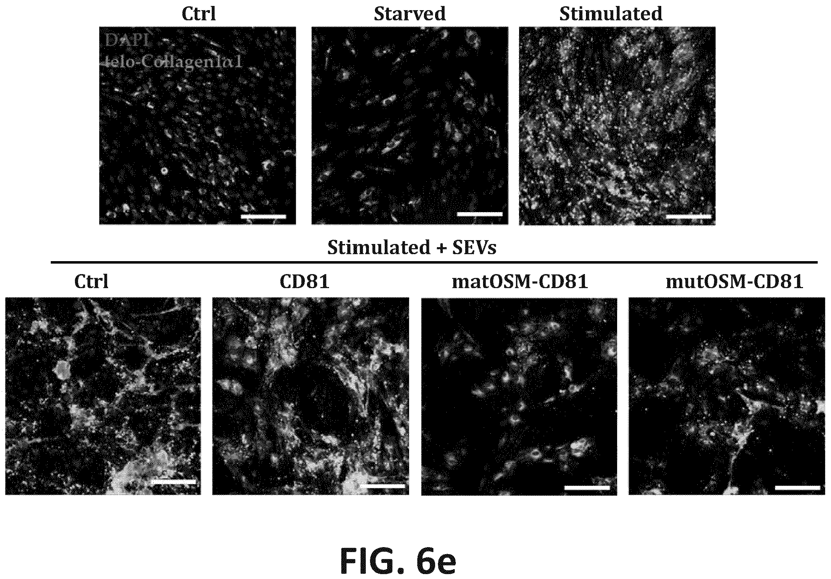

**Figure 6****. MutOSM-CD81 enriched SEVs from MSC-T showed enhanced ability to reduce HCF-V proliferation rate after starvation and teloCol1 *α* 1 expression and secretion to the extracellular space upon pro-fibrotic phenotype in vitro stimulation. (a)** Quantification of HCF-V proliferation rate under basal conditions (Ctrl), after starvation (starved) and after starvation and treatment with SEVs. Ratio between number of proliferative cells (Ki-67 positive nuclei, in green) and total number of cells (DAPI positive nuclei, in blue) is shown. **(b)** Representative pictures showing Ki-67 and DAPI nuclei staining for each experimental condition. **(c-d)** Quantification of telo-Collagen1 *α* 1 protein, showed in green. Mean fluorescence intensity normalised to number of cells (MFI) and extracellular telo-Collagen1 *α* 1 area are shown. **(e)** Representative pictures showing telo-Collagen1 *α* 1 detection (shown in green) on samples under all experimental conditions. DAPI was used for nuclei staining (shown in blue). Scale bar = 200 µm. Both starved and stimulated cells were treated with equivalent volumes of vehicle (PBS). All results were obtained from three independent experiments and were analyzed with ANOVA and Tukey's post-hoc test. All experimental conditions were compared to both starved cells and starved cells plus ctrl SEVs for statistical analysis in proliferation experiments. Stimulated cells and stimulated cells treated with ctrl SEVs were used as reference to compare the rest of conditions for fibrosis in vitro model. using values from ctrl cells or ctrl SEVs as reference conditions and represented as mean ± SEM (ns: non-significant statistic differences; *p<0.05; **p<0.01: ***p<0.001).

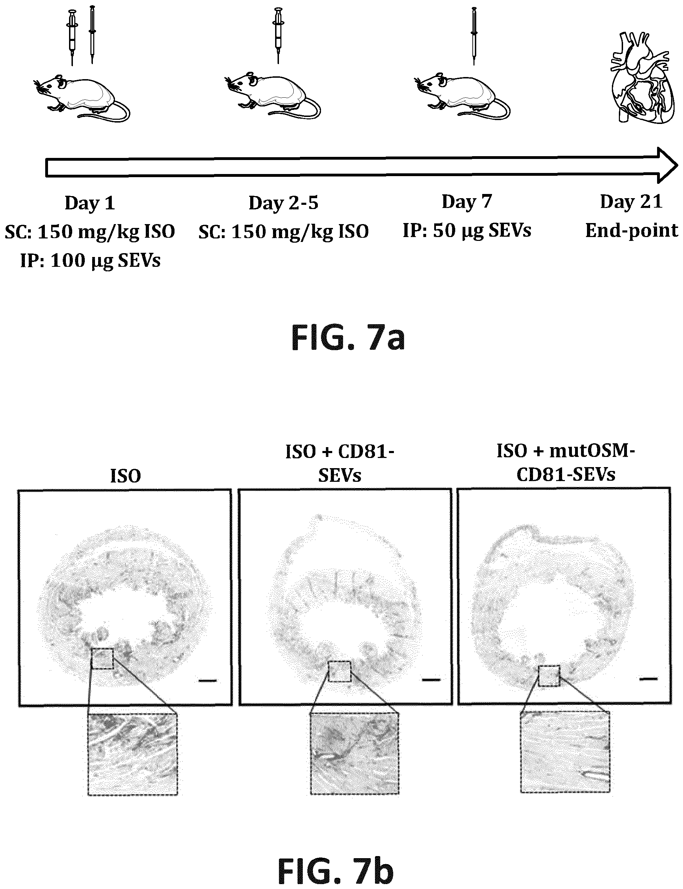

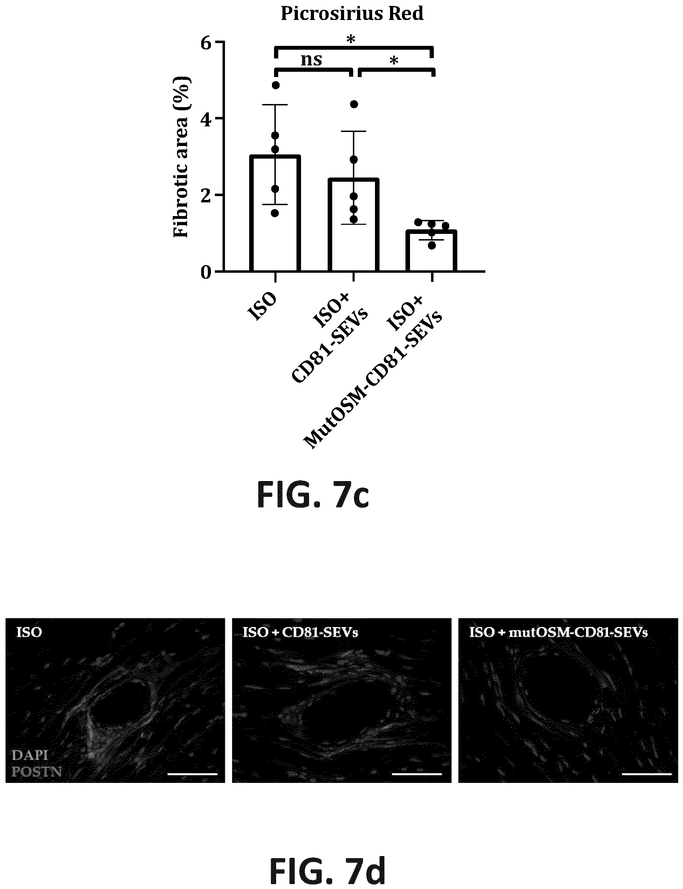

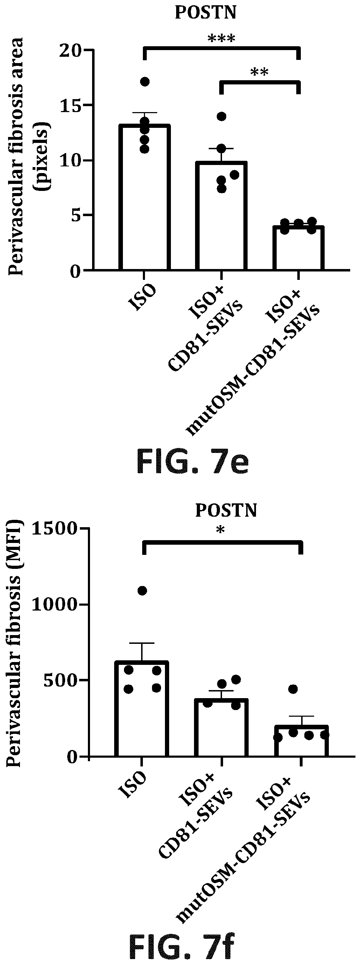

**Figure 7****. MutOSM-CD81 SEVs from MSC-T reduced collagen area after fibrosis induction in an isoproterenol (ISO) mice in vivo model. (a)** Illustration showing in vivo experimental procedure. Three experimental groups were included: ISO (ctrl), ISO + CD81-SEVs and ISO + mutOSM-CD81-SEVs Fibrosis was induced by a daily isoproterenol subcutaneous (SC) injection (dose: 150 mg/kg) during 5 consecutive days. Two intraperitoneal (IP) doses of SEVs (CD81 or mutOSM-CD81) were injected (day 1 and day 7). An equivalent volume of vehicle (PBS) was injected in ISO experimental group. **(b)** Representative pictures of picrosirius red staining. Scale bar: 200 µm. **(c)** Quantification of fibrotic area in Picrosirius Red stained heart sections. Percentage of collagen covered area related to total heart area is represented for each animal. **(d)** Representative pictures of periostin (POSTN) staining. Scale bar: 50 µm. **(e-f)** Quantification of perivascular fibrosis by POSTN staining in pixels and mean fluorescence intensity. Five animals were included in each group. ImageJ was used for image analysis and colorimetric and fluorescence quantification. Data were analysed with ANOVA and Tukey's post-hoc test using values from ctrl group (ISO) as reference conditions and represented as mean ± SEM. Data from CD81-SEVs and mutOSM-CD81-SEVs were also compared (ns: non-significant statistic differences; *p<0.05; **p<0.01: ***p<0.001).

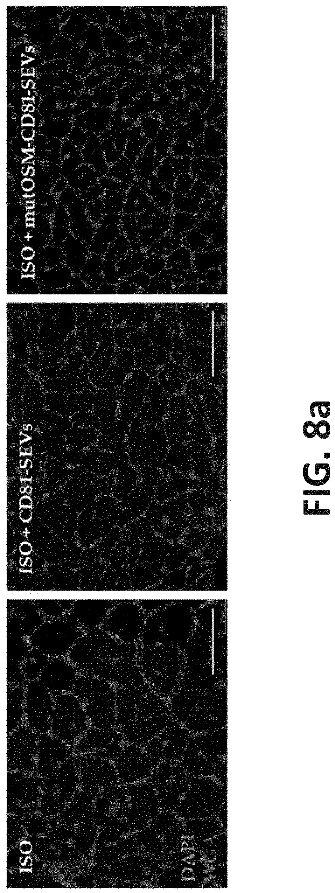

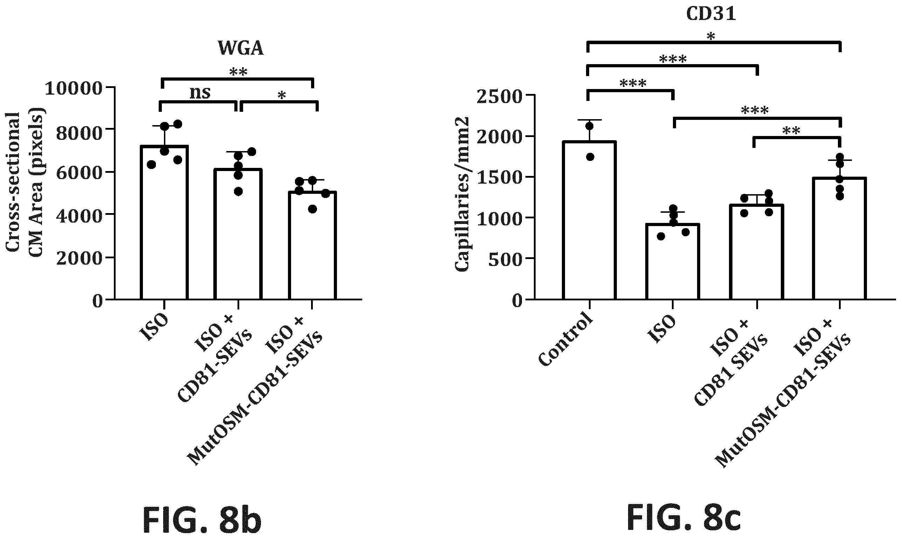

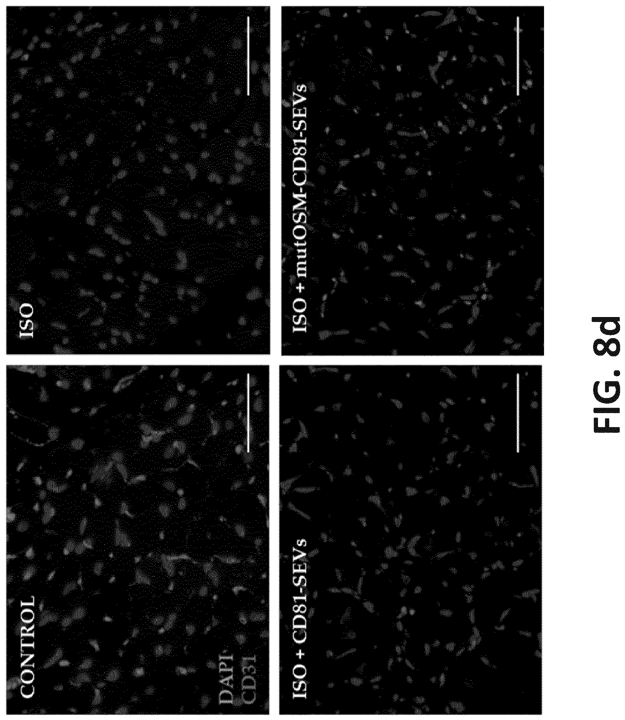

**Figure 8****: MutOSM-CD81 SEVs from MSC-T reduced cardiac hypethrophy and increased angiogenesis in an isoproterenol (ISO) mice in vivo model.** (a) Representative pictures of WGA staining (shown in red) and nuclei staining (DAPI, shown in blue) for each experimental group. Scale bar: 50 µm. **(b)** Measurement of cardiomyocytes area using wheat germ agglutinin (WGA) staining in heart sections of animals of different groups. **(c)** Measurement of microvasculature using CD31 antibodies . Circular stained structures with an area up to 23.5 µm² were counted. **(d)** Representative pictures of CD31 staining (shown in red) and nuclei staining (DAPI, shown in blue) for each experimental group. Scale bar: 50 µm. Five animals were included in each group. ImageJ was used for image analysis and colorimetric and fluorescence quantification. Data were analysed with ANOVA and Tukey's post-hoc test using values from ctrl group (ISO) as reference conditions and represented as mean ± SEM. Data from CD81-SEVs and mutOSM-CD81-SEVs were also compared (ns: non-significant statistic differences; *p<0.05; **p<0.01: ***p<0.001).

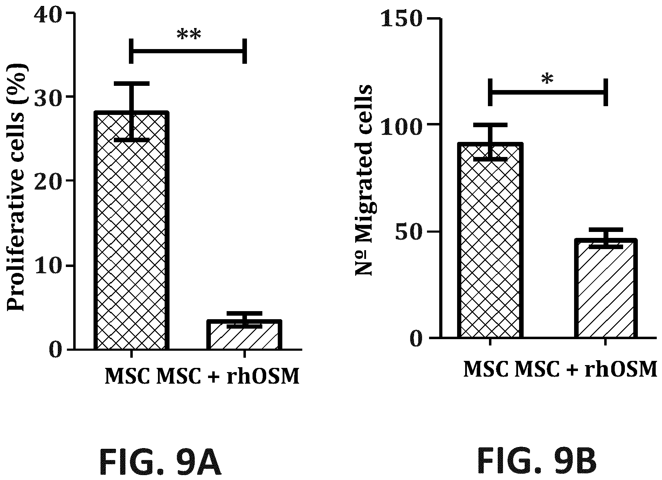

**Figure 9****: Human recombinant OSM reduced MSC proliferation in vitro. (a)** Percentage of proliferative cells untreated (MSC) or treated with rhOSM (MSC+rhOSM, dose: 10 ng/mL) after BrdU assay. **(b)** Number of migrated cells in basal conditions (MSC) and after rhOSM treatment (MSC+rhOSM, dose: 10 ng/mL) measured by transwell assay. Two independent experiments were included. Unpaired t-test was used to compare the means. Asterisks represent statistically significant differences (*p<0.05; **p<0.001). Mean±SEM for each data set is represented.

### Detailed description of the invention

Based on the existing needs and problems in the prior art, the present invention provides Small Extracellular Vesicles (SEVs) containing functional Oncostatin M (OSM) to modulate pro-fibrotic diseases.

In the context of the present invention the authors have surprisingly found the feasibility to load a soluble cytokine, such as OSM, on SEVs, secreted by mesenchymal stromal cells (MSCs), by modifying a unique amino acid of its sequence. In addition, the authors have also found that genetically modified cells and SEVs do not show changes in their properties after introducing the new recombinant properties compared to control samples.

The authors further unexpectly found that this genetic modification provides a greater anti-fibrotic capacity to the vesicles secreted by mesenchymal stromal cells (MSCs) with respect to vesicles derived from native MSCs.

This modification of the content of the SEVs is achieved through the genetic modification of an MSC line that allows the insertion of the OSM into the SEVs in a specific way.

The strategy to introduce OMS into the vesicle membrane can be achieved with any type of MSC, regardless of its tissue origin. Therefore, MSCs can be isolated from different sources such as bone marrow, dental pulp, fat tissue, umbilical cord, peripheral blood, Warton gelly, etc. Preferably, SEVs of the present invention are isolated from human mesenchymal stromal cells derived from dental pulp.

The mutated OSM is stably overexpressed in the SEVs by modification of the MSCs using lentiviral technology. Specifically, SEVs are obtained using immortalized MSC cell line with human TERT (MSC-T). This cell line is genetically modified to release a mutant variant of OSM, loaded on SEVs surface, through its fusion to the tetraspanin CD81, that is naturally enriched on MSC-SEVs membrane. In addition, an inducible expression system that allows expressing the mutated Oncostatin M only in the presence of doxycycline is incorporated. This genetic modification provides a greater anti-fibrotic capacity to the vesicles secreted by MSCs with respect to vesicles derived from native MSCs.

In a first aspect, the present invention thus relates to an isolated Small Extracellular vesicle (SEVs) with a mutated form of functional Oncostatin M expressed on its surface (SEV of the invention).

In the context of the present invention, Small extracellular vesicles (SEVs) refer to small membrane particles between 30 and 250 µM derived from MSC. They are small lipid bilayer-bound vesicles released from living cells into the extracellular environment. SEV biogenesis implies the formation of specialized intracellular compartments (called multivesicular bodies or late endosomes), which is a key sorting point in the endocytic pathway. The inward budding of multivesicular bodies membrane produce numerous intraluminar vesicles that are expelled into the extracellular environment when the multivesicular bodies fuse with the plasma membrane, referred to as SEV. They lack functional nuclei and cannot replicate, which makes them an interesting alternative to the use of cells for therapeutical applications since no teraroma can be formed when administered *in vivo* and none immunogeneicity has been observed.

In the present invention, the loading of a specific protein (OSM) in SEV surface to activate a specific signalling pathway on target cells confers a method to specifically trigger a reaction on cells that are expressing the specific receptors for OSM binding (active targeting). Additionally, SEV loaded with OSM can be uptaken by the cells by traditionally described mechanisms, enhancing the native therapeutic properties of MSC-SEVs.

In a particular embodiment, to achieve the loading of OSM in SEVs, two mutated versions of OSM (MutantOSM and MatureOSM) were designed, where a punctual mutation to substitute an arginine (R) for a glycine (G) on the proteolytic cleavage site between the main chain and propeptide (PP) domains of the OSM sequence was included.

MutantOSM (mutOSM), of sequence SEQ ID NO 1, consists of the full sequence of human OSM but introducing a punctual mutation to substitute an arginine (R) for a glycine (G) on the proeloytic cleavage site between the main chain and propeptide (PP) domain of the sequence. MatureOSM (matOSM), of sequence SEQ ID NO 2, contained the same punctual mutation, but not the PP domain.

All OSM versions are fused to N-terminal human CD81 to maintain modified OSM on the outer part of SEVs membrane.

In a second aspect, the present invention thus also refers to a genetic construct comprising a DNA recombinant sequence comprising the modified OSM sequence, selected from SEQ ID NO 1 or SEQ ID NO 2, fused to CD81 sequence (mutOSM-CD81, of sequence SEQ ID NO 3 and matOSM-CD81, of sequence SEQ ID NO 4, respectively) (genetic construct of the invention).

The authors of the present invention have shown that the expression of OSM in MSCs reduces their migratory capacity and their proliferation rate, therefore, the overexpression of OSM inhibits the cellular expansion of the MSCs to produce the therapeutic agent. However, in the present invention this effect has been controlled by introducing an inducible expression system based on the use of doxycycline as an inducer of the expression of the fusion protein, formed by the mutated form of OSM, to make it resistant to intra and extracellular lysis, and CD81, whose expression allows the secretion of the OSM in the SEVs. Therefore, genetically modified cells of the present invention have a proliferation rate similar to that of native MSCs and their proliferation is only decreased when doxycycline is added to the culture medium of the genetically modified MSCs.

According to the above, in a preferred embodiment, the expression of the genetic construct of the invention is controlled by a Tetracycline-controlled transcriptional activation method (Tet-On system), with an inducible promoter, which responds to doxocycline.

Therefore, in a third aspect, the present invention also refers to an expression vector (vector of the invention) comprising:
a. A genetic construct comprising a DNA recombinant sequence comprising the modified OSM sequence, selected from SEQ ID NO 1 or SEQ ID NO 2, fused to CD81 sequence (mutOSM-CD81 (SEQ ID NO 3) and matOSM-CD81 (SEQ ID NO 4, respectively) (genetic construct of the invention), and
b. An inducible promoter, which responds to doxycycline.

In a fourth aspect, the invention also refers to the genetically modified human mesenchymal stromal cells comprising the expression vector of the present invention. MSCs can be obtained from different sources such as bone marrow, dental pulp, fat tissue, umbilical cord, peripheral blood, Warton gelly, etc. Preferably, MSCs are human mesenchymal stromal cells derived from dental pulp.

In a fifth aspect, the present invention refers to an in vitro method for producing the SEVs of the invention, comprising the steps of:
a. Immortalizing a human MSC line by telomerase overexpression,
b. Introducing in the immortalized MSCs the vector expression of the invention,
c. Culturing the genetically modified MSCs obtained in b) in a medium culture under conditions permitting their expansion,
d. Adding doxocycline to the culture medium, and
e. Collecting Small Extracellular vesicles (SEVs) from the culture supernatant.

The expression of OSM inhibits the growth of the MSCs, so it is necessary to expand the cells in the absence of the expression of the OSM gene, and then use the inducible vector to generate its expression. Thus, MSCs are grown and expanded in a medium without doxycycline (c). Once a sufficient number of MSCs has been achieved to obtain the desired amount of SEVs, doxycycline is added (d) to activate the doxycycline-inducible promoter (Sistems TET-ON) which causes the cells to start expressing the OSM in their intracellular medium and also releasing it in the SEVs through OSM fused to tetraspanin CD81.

In step e) SEVs can be collected from MSCs by various methods, the most common and most preferred is differential centrifugation. Procedures that may be used for preparing SEVs ot the invention include exclusion chromatography, ionic exchange chromatography, tangential flow filtration (TFF) or a combination of any of them.

The incorporation of the mutated OSM together with the rest of the paracrine factors contained in the SEVs from the modified MSCs presents additional advantages. On the one hand, the integrity and stability of the OSM is protected, since the supply of its soluble form is more susceptible to degradation in biological fluids. Additionally, the protection of the functionality of the molecule increases the effective dose and requires the use of a lower concentration than if it were administered freely. On the other hand, by isolating and modifying the SEVs, the molecules of interest can be directed, together with the rest of the contents of the MSC-derived SEVs, to the target cells in a more specific way, reducing the side effects derived from the administration of the free molecule.

The SEVs of the invention present improved antifibrotic properties, preventing cardiomyocytes hypertrophy, reducing fibrosis and showing a potent angiogenic effect. Therefore, the use of SEVs in medicine allows the development of important clinical applications in the field of treatment and prevention of fibrotic diseases.

The invention thus also relates to a pharmaceutical composition comprising one or more EVs of the invention as active ingredient. Pharmaceutical compositions of the invention can additionally comprise a pharmaceutically acceptable excipient or carrier.

Examples of such carriers and excipients include tonicity adjusting agents, thickeners, saccharides, sugar alcohols, antiseptics (preservatives), bactericides or antibacterial agents, pH adjusters, stabilizers, chelating agents, oleaginous bases, gel bases, surfactants, suspending agents, bonding agents, excipients, lubricants, disintegrants, foaming agents, fluidizing agents, dispersants, emulsifiers, buffers, solubilizing agents, antioxidants, sweeteners, acidulating agents, colorants, flavoring agents, perfumes, and cooling agents, but not limited thereto.

An administration route of the SEVs or pharmaceutical compositions comprising the SEVs of the invention includes oral administration, subcutaneous administration, intramuscular administration, intravenous administration, intraarterial administration, intrathecal administration, and intraperitoneal administration, depending on the dosage form.

It will be clear to the skilled artisan that when describing medical applications of the SEVs, the present invention normally relates to a plurality of nanovesicles, i.e. a population of SEVs which may comprise thousands, millions, billions or even trillions of SEVs. In other words, individual SEV when present in a plurality constitute an SEVs population. Thus, naturally, the present invention pertains both to individual SEV and populations comprising SEVs, as will be clear to the skilled person. The dosages of SEVs when applied in vivo may naturally vary considerably depending on the disease to be treated, the administration route, the therapeutic activity, effects, any targeting moieties present on the SEVs, the pharmaceutical formulation, etc. Furthermore, the SEVs of the present invention may also comprise additional therapeutic agents.

In another aspect, according to the above, the invention refers to the use of the SEVs of the invention, or the pharmaceutical composition comprising the SEVs as described herein, in the treatment or prevention of fibrotic diseases.

In the context of the present invention fibrotic diseases refer to those diseases that promote interstitial fibrosis leading to organ disfunction, such as cardiac, pulmonary or hepatic fibrosis.

In a preferred embodiment, the fibrotic disease is myocardial infarction. Type 1 myocardial infarction occurs in those with atherosclerotic plaque rupture and thrombosis, and is characterized by transmural fibrosis, whereas type 2 myocardial infarction occurs due to myocardial oxygen supply and demand imbalance in the context of an acute illness causing tachyarrhythmia, hypoxia, or hypotension without acute atherothrombosis, and is characterized by diffuse fibrosis.

In a more preferred embodiment, the invention refers to the use of the SEVs of the invention, or a pharmaceutical composition comprising the SEVs, to prevent or revert the diffuse fibrosis ocurred in Type 2 myocardial infarction.

The following examples illustrate the present invention:

### EXAMPLES

### Material and methods

### Prokaryote cell types

Douglas Hanahan 5 α^{™} (DH5α) Competent Cells were obtained from Thermo Fisher Scientific (Waltham, MA, USA). Bacteria genotype was "F- Φ80lacZΔM15 Δ(IacZYA-argF) U169 recA1 endA1 hsdR17(rk-, mk+) phoA supE44 thi-1 gyrA96 relA1 λ- ". Colony selection after bacteria transformation was performed using ampicillin antibiotic (Thermo Fisher Scientific, Waltham, MA, USA).

### Eukaryotic cell lines

Human embryonic kidney (HEK 293T) cell line was obtained from the American Type Culture Centre (ATCC, Manassas, VA, USA). HEK293T were cultured in high glucose DMEM supplied with 10% of heat inactivated foetal bovine serum (FBS) and 1% of penicillin/streptomycin solution (P/S), all from Gibco (Thermo Fisher Scientific, Waltham, MA, USA). Dental pulp mesenchymal stromal cells (MSC) in passage 1/2 were obtained from the Spanish National Cell Line Bank (BNLC) through Inbiobank Foundation (San Sebastián, Spain. The same biopsy of MSC was immortalized and characterized in our laboratory through telomerase reverse transcriptase lentiviral transduction (MSC-T) (Gómez-Ferrer, M. et al. Hif-1a and proinflammatory signaling improves the immunomodulatory activity of MSC-derived extracellular vesicles. Int J Mol Sci 22, (2021*)).* MSC and MSC-T were cultured in low glucose DMEM supplied with 10% of heat inactivated FBS and 1% P/S, all from Gibco. Human Ventricular Cardiac Fibroblasts (HCF-V) were obtained from PromoCell and cultured in Fibroblasts Growth Medium-3 BulletKitTM (FGMTM-3; PromoCell, Heildelberg, Germany), following manufacturer's instructions. All cellular types were cultured in a Forma Series II Incubator Model 3141 (Thermo Fisher Scientific, Waltham, MA, USA).

### OSM plasmids design

Fusion proteins for MSC-T genetic modification were designed to anchor two versions of OSM to CD81 tetraspanin. MutantOSM (mutOSM) consisted of the full sequence of human OSM but introducing a punctual mutation on the proteolytic cleavage site between the main chain and propeptide (PP) domains of the sequence, resulting in a substitution of an arginine (R) by a glycine (G). Mature OSM (matOSM) version contained the mutation, but not the PP domain, while mutant OSM contained the full sequence of OSM, maintaining the previous specified mutation. All OSM versions were fused to N-terminal human CD81 to maintain modified OSM on the outer part of small extracellular vesicles (SEVs) membrane. A third plasmid containing only CD81 was included. The expression of CD81, matOSM-CD81 and mutOSM-CD81 was controlled by a Tet-On inducible promoter, which responds to doxycycline. Green fluorescent protein (GFP) was included in the three vectors, and its expression was controlled by a constitutive promoter (EF1). Plasmids were obtained from SBI Gene Universal (Gene Universal Inc., Newark, Denmark).

### Bacteria transformation and amplification

Plasmids were transformed and amplified in DH5α^{™} bacteria using standard heat shock protocols. Briefly, bacteria were mixed with the correspondent DNA plasmids and bacteria were transformed in a pre-warmed water bath (42 °C) for 45 seconds and placed on ice for 2 minutes. Then, 1 mL of pre-warmed lysogeny broth medium (LB; Sigma Aldrich, MO, USA) was added and samples were incubated at 180 rpm in an orbital shaker at 37 °C for 1h. Samples were centrifuged at 400 g for 1 min and pellet was resuspended in 100 uL LB. The resulting mixture was added to LB-agar Petri dishes with ampicillin for bacterial clone selection and incubated in a bacteriological incubator overnight.

### Isolation and purification of plasmid DNA from bacteria

Colonies were individually picked in tubes containing LB with ampicillin and incubated in an orbital shaker at 180 RPM and 37 °C for 6 to 8 h. Plasmid MiniPrep Kit (GE Healthcare, Chicago, IL, USA) was used for DNA isolation from bacteria following manufacturers instructions. For larger amounts of DNA, transformed bacteria were further grown into flasks with 200 mL LB containing ampicillin at 180 RPM and 37 °C overnight, and Jetstar Plasmid Maxi Prep Kit (Gentaur, Malaga, Spain) was used DNA isolation. DNA was quantified by spectrophotometry (NanoDrop 1000, Thermo Fisher Scientific, Waltham, MA, USA).

### Engineered MSC-T generation

Genetically modified MSC-T for CD81, matOSM-CD81 and mutOSM-CD81 overexpression contained a Tet-On inducible vector system whose expression depends on the presence of doxycycline (10 µg/mL; from Sigma-Aldrich, MO, USA). Lentiviral transduced MSC-T were seeded under the same conditions, but standard FBS was replaced for heat inactivated tetracycline-free FBS (BioWest, Nuaillé, France) to allow the expression of the vector encoding proteins only when doxycycline was added to cell culture medium. HEK293T cells were used as packaging cell line to produce lentiviral particles using standard lentiviral transduction protocols (Gándara, C., Affleck, V. & Stoll, E. A. Manufacture of Third-Generation Lentivirus for Preclinical Use, with Process Development Considerations for Translation to Good Manufacturing Practice. Hum Gene Ther Methods 29, 1-15 (2018*)).* After titration, an adequate MOI of viral particles was used to transfect MSC-T, and 8 µg/mL polybrene (Sigma Aldrich, MO, USA) was added to increase infection rate.

### SEVs isolation

SEVs recollection medium (RM) contained low glucose DMEM culture medium with 10% vesicles-free FBS and 1% P/S. Doxycycline (Dox; Sigma Aldrich, MO, USA) was added at a final concentration of 10 µg/mL and refreshed every day. Vesicles naturally present in FBS were removed by ultracentrifugation at 15,0000 g for 16 h at 4 °C followed by filtration through polyether sulfone membrane filter with a pore size of 0.22 µm as described by C. Théry et al. *(*Théry, C., Amigorena, S., Raposo, G. & Clayton, A. Isolation and Characterization of Exosomes from Cell Culture Supernatants and Biological Fluids. Curr Protoc Cell Biol 30, 3.22.1-3.22.29 (2006*)).* Cells were incubated in SEVs RM with Dox for 48 h before supernatant collection for SEVs isolation.

SEVs isolation from RM was performed by serial ultracentrifugation and filtration steps. All steps of this protocol were performed at 4 °C. First, supernatant was centrifuged at 2,500 g for 20 min to precipitate and remove remaining cells. Supernatant was transferred to ultracentrifuge tubes and centrifuged at 15,000 g for 45 min to rush the bottom cellular debris. Then, supernatant was again collected and filtered (pore size 0.2 µm) to exclude large EVs. Thereafter, filtered supernatant was transferred to clean tubes and ultracentrifuged at 100,000 g for 2 h. Then, supernatant was completely removed, and each pellet was resuspended in 1 mL of cold PBS. Resulting solutions from the same biological conditions were pooled in only one tube for a final washing step, which was achieved by a second ultracentrifugation step at 100,000 g for 2h. Finally, supernatant was removed and SEVs pellet was resuspended in the desired solution.

Protein content on isolated SEVs simples was measured by BCA assay following manufacturers' recommendations (PierceTM BCA Protein Assay Kit, Thermo Fisher Scientific, Waltham, MA, USA). The dose of SEVs protein used for in vitro experiments was 30 µg/mL.

### Transmission electron microscopy (TEM)

SEVs pellets collected from equal amounts of culture medium were suspended in 200 µL of PBS, loaded onto pure carbon coated copper grids, fixed with 2% PFA and 1% Glutaraldehyde, contrasted with 1% Uranyl acetate and embedded in 0.4% methyl cellulose to analyse by negative staining. The grids were examined with a transmission electron microscope FEI Tecnai G2 Spirit (Thermo Fisher Scientific, Waltham, MA, USA). All images were acquired using a digital camera Morada (Olympus Soft Image Solutions GmbH, Münster, Germany).

### Nanoparticle tracking analysis (NTA)

NTA was used to determine SEV concentration and particle size distribution. All particle tracking analyses were performed using a LM14C Nano Sight instrument (Malvern Instruments Ltd, Malvern, UK) using the same settings (camera level 16, 3 videos of 90 s and 1300/512 slider shutter/gain, respectively) at the proper concentration to obtain around 50 particles/frame. Analysis of the acquired videos was performed with threshold 5 and gain 12 with NTA3.2 program.

### Single particle fluorescence and interferometry imaging with ExoView^{®}

Isolated SEVs from the different genetically modified MSC were diluted 1:10 in Incubation solution (1X). 50 µL of this sample was carefully pipetted onto the silicon chip coated with individual antibody spots against mouse CD9, CD63, and CD81 as well as negative isotype controls. After overnight incubation in a 24-well plate, chips were washed three times on an orbital shaker orbital at 500 RPM for 3 minutes with Solution A. Then, the chips were incubated on orbital shaker at 500 RPM for 1 hour at RT with a cocktail of fluorescent antibodies (anti-CD8-AF488; anti-CD9-AF555; anti-OSM-AF647) diluted in Blocking solution. Chips were washed once in Solution A, three times in Solution B, and once in deionized water. Chips were carefully removed from the 24-well plate, washed further in deionized water and removed for drying. Image and data acquisition for each chip was performed with the ExoView^{®} R100 (NanoView Biosciences). Data analysis was performed with ExoView Analyzer 3.1.4 (NanoView Biosciences). Oncostatin M antibody was purchased from ThermoFisher (Cat# # MA5-23810, Clone: 17001). Fluorescent conjugation was performed with Alexa Fluor^{®} 647 Conjugation Kit (ab269823).

### HCF-V fibrotic phenotype stimulation in vitro

HCF-V were seeded density of 25,000 cells/cm2 and were allowed to attach for 24 h. Therefore, culture media was replaced for starvation media (high glucose DMEM, 0.5% vesicles-depleted FBS, 1% non-essential amino acids and 1% P/S). After 16 h, fibroblasts were stimulated with 100 µM L-ascorbic acid 2-phosphate (Sigma Aldrich, MO, USA), 3.16 ng/mL ng/mL recombinant TGFβ-1 (R&D Systems, Minneapolis, MI, USA) and 3.16 µg/mL dextran sulphate (Sigma Aldrich, MO, USA) as previously described *(*Palano, G. et al. A high-content, in vitro cardiac fibrosis assay for high-throughput, phenotypic identification of compounds with anti-fibrotic activity. J Mol Cell Cardiol 142, 105-117 (2020*)).* Treatments were added at the same time than stimulation media. Unstimulated cells (cells starved but not stimulated, starved) and untreated cells (neither starved nor stimulated, ctrl) were used as control conditions. HCF-V were stimulated for 48h before proceeding to read-outs.

### Western Blotting

Protein samples from cell lysates or SEVs were separated by 10% SDS-PAGE, transferred to PVDF membranes (Thermo Scientific, Waltham, MA, USA). Thereafter, PVDF membranes were blocked with 5% dry milk in Tris-buffered saline (20 mM Tris pH 7.5, 150 mM NaCl). Primary antibodies diluted in blocking solution were incubated at 4°C overnight. The following primary antibodies were used: α-hOSM, (R&D Systems, Minneapolis, MI, USA); α-CD81 (Abcam, Cambridge, UK); α-GP130 (Cell Signaling Technology, Danvers, MA, USA); a-IL-31RA (Abcam, Cambridge, UK); α-IL-31-RB (Proteintech, Rosemont, IL, USA) and LIF-R (R&D Systems, Minneapolis, MI, USA). Antibodies were used at a 1:200 dilution. Non-bound primary antibody was removed by three washing steps with PBS solution. Blots were then incubated with IgG-HRP secondary antibodies for 1 hour at room temperature (Promega, Madison, Wl, USA) and signals were detected using the Amersham ECL Western Blotting Detection Kit (GE Healthcare, Chicago, IL, USA). Tubulin α-1A signal was used to standardize cellular protein quantities. Densitometric analysis was performed with imaged software (NIH, USA).

### Immunofluorescence

HCF-V were fixed with 4% paraformaldehyde (PFA) for 10 minutes at 37 °C. After PFA washing with PBS, cells were permeabilized with 0.2% Triton in PBS for 1 hour at RT and blocked for nonspecific epitopes with 2% BSA in PBS (both from Sigma Aldrich, MO, USA). Thereafter, samples were incubated with primary antibodies solution, containing 0.1% BSA in PBS. The following antibodies were used: α-Ki-67 and α-teloCollagen1α1 (both from Abcam, Cambridge, UK; 1:100 dilution). Next, samples were washed 3 times with PBS and incubated with α-Rabbit Alexa Fluor 488 secondary antibody (Life technologies, CA, USA) and DAPI for 1 hour at RT. Images were taken using CellVoyager CV8000 high-throughput screening system (Yokogawa, Tokyo, Japan), and fluorescent signal was analysed with Leica Application Suite Version 2.4.0 R1 version and Image J software (NIH).

### Bromodeoxyuridine cell proliferation assay

Bromodeoxyuridine (BrdU) is a pyrimidine-analogue synthetic nucleotide which can be used to label nascent DNA in viable cells. During the process of DNA replication, BrdU can replace thymidine and incorporate into the synthesized DNA of actively dividing cells.

Firstly, cells were seeded in 18 mm coverslips in 24 multi-well plates in low-glucose DMEM supplemented with 10% FBS TTC free, 1% P/S at a density of 50.000 cells/cm2. Next day, BrdU reagent (10 µg/mL, Life Technologies) was diluted in cell culture medium containing the correspondent treatments and incubated for 8 h. After this time, cells were washed 3 times with PBS and fixed with ice-cold 70% EtOH at 4 °C for 10 min. Coverslips were washed 3 more times with PBS and incubated with 1N hydrochloric acid (HCl) at 37 °C for 30 min. HCl was used to unmask BrdU incorporated in newly synthetized DNA. For acid neutralization, borate buffer (nM: 100 boric acid, 75 sodium chloride, 25 sodium tetra-borate in distilled water; pH adjusted to 8.3) was added and samples were incubated at RT for 10 min. Anti-BrdU (Abcam, Cambridge, UK) 1:100 was used to detect BrdU incorporation by the cells. Pictures of random fields were taken using an upright fluorescence microscope (Model DM6000B, Leica) and a total of 100 nucleus were counted in each sample. Proliferation rate was calculated as the ratio of double stained DAPI and BrdU number of cells (replicative cells) and single stained DAPI cells (non-replicative cells).

### Migration assay

Cells were seeded in 24 well plate transfer inserts (pore size: 8 µm, BD Falcon) in basal culture medium (low DMEM + 0.5% FBS TTC free + 1% P/S) at a density of 10.000 cells/cm2. After cells stabilization, lower chamber medium was replaced for basal culture medium supplemented rhOSM (10 ng/mL). Basal medium was used as migration negative control. After 8 h, non-migrated cells were removed from the upper side of the transwell inserts with cotton swabs and cells in the lower part of the transwell insert were fixed with ice-cold 70% EtOH for 10 min at 4 °C. After that, membranes were washed with PBS, stained with 1 µg/mL DAPI, cut and transferred to microscope slides (Menzel Gläser, Afora). Pictures were taken in an upright fluorescence microscope (Model DM6000B, Leica) and number of nucleus on each sample were counted.

### Isoproterenol in vivo model

All experimental procedures involving the use of animals were approved by the institutional ethical and animal care committees according to guidelines from Directive 2010/63/EU of the European Parliament on the protection of animals used for scientific purposes, enforced in Spanish law under Real Decreto 1201/2005. GVA authorised procedure 2021/VSC/PEA/0214 for mice's model of isoproterenol-induced heart failure. Experimental procedure is illustrated in Figure 5a. Adult C3H/HeNCrl male mice strain (10-14 weeks old, weighting 35-40 g) were selected for in vivo studies (Charles River Laboratories, Wilmington, MA, USA). Mice were randomly distributed in three experimental groups (n=5 in each group): control (ISO), mice treated with CD81-SEVs (ISO + CD81-SEVs) and mice treated with mutOSM-CD81-SEVs (ISO + mutOSM-CD81 SEVs). Isoprotenerol (I5627, Sigma Aldrich, MO, USA) was subcutaneously infused once a day for 5 consecutive days, using a dose of 150 mg/kg/day). Intraperitoneal injection of 100 µg of SEVs protein was performed just after the first isoproterenol injection. One week later, mice received a second intraperitoneal SEVs injection (50 µg of SEVs protein). Equivalent volumes of PBS were injected in ISO group. Euthanasia was conducted after 21 days, and cardiac tissues were resected for further analysis.

### Histology

Mice hearts were harvested at day 21 after isoproterenol treatment, fixed in 2% PFA, embedded with paraffin and sectioned into 5 µm sliced for histological analysis by picrosirius red and wheat germ agglutin (WGA) staining. One part of tissue sections was stained for 30 min in a solution of 0.1% Sirius red in saturated aqueous picric acid for collagen bundle staining, as described by Junqueira et al. (Junqueira, L. C. U., Bignolas, G. & Brentani, R. R. Picrosirius staining plus polarization microscopy, a specific method for collagen detection in tissue sections. Histochem J 11, 447-455 (1979*)*. Samples were prepared and images captured with a Leica DMD108. imaged was used for whole collagen area quantification. A second batch of tissue sections were stained with 1 µg/mL WGA conjugated to Texas Red-X (Invitrogen, Thermo Fisher Scientific, Waltham, MA, USA). After that, nuclei were stained using mountain medium containing DAPI (FluorSave, CalbioChem, CA, USA). Images were captured with a Leica DM2500 fluorescence microscope (Leica Microsystems, Wetzlar, Germany) and analysed in imaged (NIH, USA), where cross-sectional area of cardiomyocytes was measured. Vessel density of cardiac tissue was assessed by staining tissue sections with α-CD31 antibody (Santa Cruz Biotechnology, Dallas TX, USA). Heat-induced antigen retrieval was conducted prior antibody labelling. Images were captured with a Leica DM2500 fluorescence microscope (Leica Microsystems, Wetzlar, Germany) and analysed in imaged (NIH, USA), where the number of vessels/mm² was measured.

### Statistical analysis

Three biological replicates (n=3) were included for all data showed on in vitro studies. Five animals were included on each experimental group for in vivo isoproterenol model. Data from all experimental groups were compared using ANOVA followed by Tukey's post hoc tests. All results are presented as mean ± SEM. Asterisks indicate statistically significant different between experimental conditions, where p<0.05 is indicated by *; p<0.01 by ** and p<0.001 by ***. Differences were considered at p<0.05 with a 95% confidence interval. All statistical analysis were performed using GraphPad Prism 8 software.

### RESULTS

### 1. Generation of MSC-T overexpressing a modified version of OSM on secreted SEVs membrane

OSM is a cytokine composed by three domains: signal peptide (SP), main chain and propeptide (PP). To be secreted as a mature protein (main chain) to the extracellular space by the producer cells, OSM needs to be enzymatically processed on the producer cells in two cleavage sites (CS), one between SP and main chain and other between the main chain and the PP (Figure 1a). First, the feasibility of loading native OSM protein in SEVs through its fusion to CD81 was evaluated. To achieve this, MSC-T were transfected with two plasmids, one containing the CD81 sequence and the other containing OSM native sequence fused to CD81 under an inducible promotor (Tet-on) since the expression of the cytokine OSM restrain MSC and fibroblasts proliferation (Abe, H. et al. Macrophage hypoxia signaling regulates cardiac fibrosis via Oncostatin M. Nature Communications 2019 10:1 10, 1-10 (2019*) (***Figure 9**). As shown in **figure 2****,** OSM protein was not detected by WB assay in SEVs from MSC-OSM-CD81 at the expected molecular weight for the fusion protein OSM-CD81 (around 47 kDa). Endogenous expression of OSM was detected in the three samples of MSC-T (control, MSC-T-CD81 and MSC-T-OSM-CD81. However, in MSC-T-OSM-CD81, OSM expression was detected at its native molecular weight (25 kDa), but not at the molecular weight expected for the recombinant protein, indicating the cleavage of OSM from CD81 under physiological conditions.

To overcome this problem and to achieve the loading of OSM-CD81 in SEVs, two mutated versions of OSM were designed, where a punctual mutation to substitute an arginine (R) for a glycine (R) in the enzymatic CS2 was included (**Figure 1b****-c**). This mutation is located between the main chain and the PP. The first version contained OSM sequence lacking PP sequence (matOSM, **Figure 1b**), while the second version included the full sequence of OSM (mutOSM, **Figure 1c**). In both plasmids, CD81 sequence was introduced at the N-terminal site of the modified OSM sequences.

After lentiviral transduction, genetically modified MSC-T variants (CD81, matOSM-CD81 and mutOSM-CD81) were expanded, and released SEVs were isolated and characterized. Non-transfected MSC-T were included in the experiments as controls. Schematic representation of the experimental process is shown in **Figure 1d****.** Transfection rate was evaluated measuring GFP expression by flow cytometry (**figure 3a**)**.** More than 87% of the MSC-T transfected with the different plasmids were positive for GFP, and GFP+ percentage of cells was maintained above 85% during the passages when cells were used for experiments in the three cell lines generated (**Figure 3b**)**.** Evaluation of MSC markers profile stablished by Mesenchymal and Tissue Stem Cell Committee *(*Dominici, M. et al. Minimal criteria for defining multipotent mesenchymal stromal cells. The International Society for Cellular Therapy position statement. Cytotherapy 8, 315-317 (2006*)* showed that MSC-T transfection did not modify MSC identity **(****Figure 3c****).** Regarding SEVs isolation and expansion, MSC-T, MSC-T-CD81, MSC-T-matOSM-CD81 and MSC-T-mutOSM-CD81 were expanded and incubated in SEVs RM containing 10 µg/mL doxycycline for 48h (**Figure 1d****,** Step 2). Collected media from cell cultures was used for SEVs isolation by serial ultracentrifugation and filtration steps (Step 3), and cells were used to obtain protein samples for further analysis. After that, the presence of recombinant proteins in the new generated cell lines and SEVs isolated from their supernatant was evaluated. **Figure 4a** shows a representative Western Blot results for OSM detection on SEVs and cells protein samples. Results confirmed that matOSM-CD81 and mutOSM-CD81 recombinant proteins were present on in cell extracts and SEVs , where a band around 40 to 45 kDa was observed in comparison to both ctrl and CD81-SEVs and cell lysates.

SEVs isolated from MSC-T, MSC-T CD81, MSC-T matOSM-CD81 and MSC-T mutOSM-CD81 RM were further characterized by electron microscopy and nanoparticle tracking analysis (NTA). Electron microscopy pictures showed that the four SEVs samples presented the expected convex morphology (**Figure 4b**)**.** NTA analysis revealed a similar size distribution in all samples, where the most majority of particles ranged on a size of 90 to 150 nm (Figure 4c and d). Moreover, particles concentration was similar in all the samples (Control-SEVs: 7,62·108 ± 4,8·107 particles/ml; CD81-SEVs: 9,71·108 ± 4,93·107 particles/ml; MatOSM-CD81-SEVs: 1,12·109 ± 1,26·108 particles/ml and MutOSM-CD81-SEVs: 9,18·108 ± 1,08·108 particles/ml).

Isolated EVs were also analysed by ExoView for immunophenotyping of different SEV subpopulations at the single-vesicle level. ExoView technology is based on single-particle interferometric reflectance imaging detection of SEVs captured on silicon substrate chip, constituted by an array of spots printed with different antibodies. This can be combined with EV immunostaining with fluorophore-conjugated antibodies, followed by fluorescence imaging, and multiplexed for the simultaneous detection of up to three distinct fluorophores (Silva A et al JEV 2021 DOI: 10.1002/jev2.12130). SEV were captured from MSC-T, MSC-T CD81, MSC-T matOSM-CD81 and MSC-T mutOSM-CD81 RM, and immobilized on the silicon substrate array functionalised with an antibody in different spots for SEVs capture. Spots functionalised with IgG isotype were used as control of non-specific SEVs adsorption to the chip substrate, with no significant numbers of particles counted. SEVs from the three experimental conditions predominately expressed CD63 so the spots were printed with antibodies against this tetraspanin and sequestrated SEVs were co-stained with anti-OSM, anti-CD81 and anti-CD9 antibodies (**Figure 4e**)**.** SEVs captured in all spots were simultaneously immunostained with fluorophore-conjugated anti-OSM or anti-tetraspanins antibodies (CD81, CD9) to visualize the pattern of SEV populations in the different experimental groups. The percentage of CD63/CD81 and CD63/CD9 double positive SEVs did not change among the experimental groups, indicating that genetic engineering of MSC-T did not influence the type of particles released by parental cells. However, as expected, a small proportion of SEVs expressed OSM only in matOSM-CD81 and MSC-T mutOSM-CD81 experimental groups (**Figure 4e**)**.** When analizing fluorescence intensity in the different experimental groups we did not observe diferences in double positive populations CD63/CD9 and CD63/CD81 in CD81, MatOSM-CD81 and MutOSM-CD81 groups but they showed higher dispersion in this parameter in comparison to ctrl group, maybe due to a less number of passages in the later group (**Figure 4f****).** The proportion of SEV double positive for CD63 and OSM was similar in MatOSM-CD81 and MutOSM-CD81-SEVs indicating that the presence or absence of propeptide did not affect the expression of the recombinant protein on SEVs surface. The proportion of CD63 expressing SEVs, double positive and also triple positive for CD81, CD9, and OSM was visualized with circular graphs in the four experimental groups (**Figure 4g**)**.**

These results showed the feasibility to load a soluble cytokine, such as OSM, on SEVs secreted by MSC-T membrane by modifying a unique amino acid of its sequence. In addition to this, genetically modified cells and SEVs did not show changes on their properties after introducing the new recombinant proteins compared to control samples.

### 2. OSM-receptors are upregulated in human cardiac ventricular fibroblasts (HCF-V) upon in vitro starvation and TGFβ-1 stimulation

It has been previously described the potential of OSM counteracting the pro-fibrotic effect of TGFβ-1 in cardiac ischemia models (Abe, H. et al. Macrophage hypoxia signaling regulates cardiac fibrosis via Oncostatin M. Nature Communications 2019 10:1 10, 1-10 (2019*);* Huguier, V. et al. Oncostatin M exerts a protective effect against excessive scarring by counteracting the inductive effect of TGFβ1 on fibrosis markers. Scientific Reports 2019 9:1 9, 1-10 (2019*)).* OSM needs to be secreted to the extracellular space and interact with either Receptor 1 (heterodimer composed by IL-31RA and LIF-R) or Receptor 2 (heterodimer composed by IL-31RA and IL-31RB) in the target cells to trigger a functional effect. Consequently, protein levels of the three OSM receptors were evaluated in target cells (human cardiac ventricular fibroblasts, HCF-V) after starvation (starved) and after in vitro stimulation, at different time points (h: 0.5, 1, 2, 4, 24 and 48), using a pro-fibrotic cocktail composed by L-ascorbic acid 2-phosphate, dextran sulphate and recombinant TGFβ-1. Non-treated HCF-V were used as control (ctrl). Representative Western Blot and densitometry quantifications are showed in Figure 5 a-d. LIF-R protein signal was increased after starvation and after starvation and 0.5 h of stimulation compared to ctrl condition (p<0.001 and p<0.01, respectively). However, LIF-R was not detected in protein samples taken from unstimulated cells or after 1, 2, 4, 24 and 48 h of HCF-V stimulation. IL-31RA receptor levels were similar between ctrl and unstimulated cells but were significantly increased from 0.5 to 4 h after HCF-V stimulation, pointing to a quick production of this protein under these experimental conditions (p<0.001 after 0.5 and 4 h of stimulation; p<0.01 after 1 and 2h of stimulation). On the contrary, a reduction on IL-31RA protein levels were found in samples taken 24 and 48 h after stimulation compared to values obtained in basal conditions (p<0.001 in both cases). Lastly, IL-31RB protein levels were measured under the same experimental conditions. An increased IL-31RB signal was observed in ctrl and unstimulated cells compared to stimulated HCF-V at different time points (p<0.001 in all cases but after 1 h of stimulation, where p<0.01).

These results indicated that two out of three receptors involved in OSM signalling pathways activation are upregulated in starving ventricular cardiac fibroblasts and at early time points after in vitro stimulation, pointing to a susceptibility to respond to OSM presence in the extracellular space.

### 3. MutOSM-CD81 enriched SEVs decrease HCF-V proliferation after starvation

The potential impact of OSM-enriched SEVs on HCF-V proliferation after starvation was measured using Ki-67 nuclei staining. Results and representative pictures are showed in **Figure 6 a-b****.** HCF-V proliferation rate was increased after cells starvation. Similar proliferation rates to starved cells were observed when HCF-V were starved and treated with ctrl or CD81-SEVs. A slight decrease on proliferation was noticed in HCF-V treated with matOSM-CD81-SEVs, although no statistically significant differences were obtained when data were compared to untreated cells or HCF-V treated with ctrl SEVs (ns). However, treatment of HCF-V with mutOSM-CD81-SEVs significantly decreased proliferation in comparison to starved cells or cells treated with ctrl-SEVs).

Overall, these results pointed to a functional role of mutOSM modifying HCF-V proliferation after starvation in vitro, indicating that loading a modified version of OSM on MSC-T-SEVs surface might promote antifibrotic features on ventricular fibroblasts.

### 4. MatOSM and mutOSM-enriched SEVs counteract telo-Collagen1α1 expression in an in vitro model of cardiac fibrosis.

Fibrotic phenotype was monitored in HCF-V by measuring the expression of mature collagen (green). Telo-Collagen1α1 signal (in green) was quantified by two different methods: measuring mean fluorescence intensity (MFI) per number of cells (nuclei stained in blue using DAPI, **Figure 6c**) (FIGURA and the area occupied by extracellular (EC) telo-Collagen1α1 in related to the total area (**Figure 6d**). Representative images for each experimental condition are shown in **Figure 6e****.** Fluorescent signal for telo-Colllagen1α1 was barely observed in control (ctrl) and starved HCF-V compared to stimulated cells, where a visible increase of telo-Collagen1α1 expression was noticed. This observation was embodied on MFI and fluorescent area quantifications (p<0.001 for control and starved conditions compared to stimulated HCF-V). A slight decrease on telo-Collagen1α1 MFI and secretion to EC area was noticed, where ctrl-SEVs reduced both values showing statistically significant differences compared to stimulated cells (p<0.01) and CD81-SEVs only reduced significantly telo-Collagen1α1 secretion to EC compared to stimulated cells (p<0.01). Importantly, treatment of HCF-V with both matOSM-CD81-SEVs and mutOSM-CD81-SEVs reduced telo-Collagen1α1 signal and EC deposition significantly compared to stimulated cells (p<0.01 and p<0.001). No major differences were noticed when HCF-V were treated with neither matOSM-CD81-SEVs nor mutOSM-CD81-SEVs compared to CD81-SEVs in terms of MFI. However, statistically significant differences were observed in terms of EC telo-Collagen1α1 deposition (p<0.01 and p<0.001, respectively), suggesting an active role of OSM-enriched SEVs on telo-Collagen1α1 EC deposition reduction after fibrosis stimulation in vitro.

In summary, these results indicated that native MSC-T-SEVs have a certain intrinsic capacity to counteract fibroblasts activation upon stimulation and adding OSM to these SEVs can maximize this effect, making OSM a good candidate to temper cardiac fibrosis after injury.

### 5. MutOSM-CD81-SEVs MSC-T reduce cardiac fibrosis in an isoproterenol in vivo model

Functional comparison in vitro of matOSM-SEVs and mutOSM-CD81 did not show significant differences. Thus, to work according to the 3R's recommendations, only mutOSM-CD81-SEVs functionality in vivo in an isoprotenerol (ISO) model of cardiac fibrosis in mice, where CD81-SEVs were used as control treatment, was evaluated. Mice were randomly distributed in three experimental groups: ISO+CD81-SEVs, ISO+mutOSM-SEVs and control (ISO), where equivalent volumes of PBS were injected. A schematic overview of the experimental set up is shown in **Figure 7a****.** Mice were injected with ISO in 5 consecutive days, and SEVs or PBS treatments were administered at day 1 and 7. Animals were terminated on day 21 for histology analysis.

Heart sections were stained with Picrosirius red and collagen area was quantified (**Figure 7** **b**, c). No statistical differences in collagen area were observed between mice treated with CD81-SEVs and ctrl mice (ISO, ns). However, a significant reduction on collagen area was observed when mice were treated with mutOSM-SEVs compared to both ctrl mice (ISO) and CD81-SEVs treated mice (P<0.05 in both cases). The presence of myofibroblasts 21 days after the beginning of ISO treatment was determined by periostin staining and as expected, mice treated with mutOSM-CD81-SEVs showed a reduction in these cells in comparison to both CD81-SEVs and ISO groups (p<0.05, **Figure 7 d** **and** **7e-f**).

Cardiac hypertrophy was measured using wheat germ agglutinin staining (WGA) to measure average area of cardiomyocytes. These data are shown together with representative pictures for each group in **Figure 8a****,** and **Figure 8b****.** Results showed a decrease CM area in mice treated with CD81-SEVs compared to ISO group (p<0.05), and an accentuated reduced area in mice treated with mutOSM-CD81-SEVs compared to both ISO group (p<0.01) and mice treated with CD81-SEVs (p<0.05). Quantification of angiogenesis was performed using anti-CD31 staining. The results showed a potent angiogenic effect in mice treated with mutOSM-CD81-SEVs in comparison to EVs and control conditions (Figure 8c and 8d). In this case, we also observed a therapeutic effect of mutOSM-CD81-SEVs in terms of increase of vessel density in mutOSM-CD81-SEVs treated mice compared to both ISO group (p<0.01) and mice treated with CD81-SEVs (p<0.001).

Altogether, these results point to an enhanced functional effect of modified OSM on MSC-T SEVs surface compared to native MSC-T-SEVs counteracting cardiac fibrosis, as shown by a decrease in heart weight, collagen deposition area and cardiomyocytes hypertrophy.

The presence of OSM was observed by western blot in both cells and SEVs carrying the two variants of OSM (mut-OSM-CD81 and mat-OSM-CD81) compared to control SEVs. Both mutOSM-CD81-SEVs and matOSM-CD81-SEVs reduced proliferation of HCF-V after starvation and mature collagen expression after TGF-β stimulation in vitro in comparison to control SEVs. When injected intravenously in mice treated with isoproterenol, mutOSM-CD81-SEVs prevented cardiac hypertrophy and reduced fibrosis as assessed by Picrosirius red staining and periostin immunohistochemistry of heart sections.

Overall, it was demonstrated that the enrichment in functional OSM on SEVs-MSC-T increased its therapeutic potency in terms of anti-fibrotic properties both in vitro and in vivo.

## Claims

1. An isolated Small Extracellular Vesicle (SEV) with a mutated form of functional Oncostatin M expressed on its surface.

2. The SEV according to claim 1 wherein the mutated OSM is fused to CD81 sequence.

3. The SEV according to claim 1 or 2 wherein the sequence of the mutated form of OSM comprises a punctual mutation to substitute an arginine (R) for a glycine (G) on the proteolytic cleavage site between the main chain and propeptide (PP) domains of the OSM sequence.

4. The SEV according to claim 3 wherein the mutated form of OSM has the sequence of SEQ ID NO 1 (mutOSM) or SEQ ID NO 2 (matOSM).

5. The SEV, according to any of claims 1-4 derived from genetically modified mesenchymal stromal cells (MSCs).

6. The SEV according to any of claims 1-5 for use in medicine.

7. Pharmaceutical composition comprising one or more SEV, according to claims 1-5, as active ingredient.

8. The SEV according to claims 1-5, or the pharmaceutical composition according to claim 7, for use in the treatment of fibrotic diseases.

9. The SEV or the pharmaceutical composition for use according to claim 8 wherein the fibrotic disease is myocardial infarction.

10. The SEV or the pharmaceutical composition for use according to claim 9 wherein the fibrotic disease is type 2 myocardial infarction.

11. Genetic construct comprising a DNA recombinant sequence comprising a modified OSM sequence, selected from SEQ ID NO 1 or SEQ ID NO 2, fused to CD81 sequence.

12. Expression vector comprising:
a. A genetic construct according to claim 11, and
b. An inducible promoter, which responds to doxycycline.

13. Genetically modified human mesenchymal stromal cells comprising an expression vector according to claim 12.

14. An in vitro method for producing SEVs according to claims 1-5 comprising:
a. Immortalizing a human MSC line by telomerase overexpression,
b. Introducing in the immortalized MSCs a vector expression according to claim 12,
c. Culturing the genetically modified MSCs obtained in b) in a medium culture under conditions permitting their expansion,
d. Adding doxocycline to the culture medium of c), and
e. collecting SEVs from the supernatant of the culture medium of d).
